# EUROPÄISCHE PATENTANMELDUNG

(11) **EP 3 888 644 A1**
(43) Veröffentlichungstag der Anmeldung: **06.10.2021**
(21) Anmeldenummer: 20167346.4
(22) Anmeldetag: 31.03.2020
(51) Int. Cl.: A61K 31/198, A61K 33/04, A61P 5/14, A61P 35/00, A61P 37/00

(54) **KOMBINATIONSPRÄPARAT VON LEVOTHYROXIN MIT EINER SELENVERBINDUNG FÜR DIE SCHILDDRÜSENTHERAPIE**

(71) Anmelder: St. Barbara Apotheke Inh. Wolfgang Endres, 92536 Pfreimd (DE)
(72) Erfinder: ENDRES, Wolfgang, 92536 Pfreimd (DE)
(74) Vertreter: Raible Deissler Lehmann Patentanwälte PartG mbB

(57) **Zusammenfassung**

Die vorliegende Erfindung betrifft eine pharmazeutische Zusammensetzung, welche das Schilddrüsenhormon L-Thyroxin und eine Selenverbindung im Gewichtsverhältnis von 2:1 bis 1:2 enthält, zur Verwendung als Medikament. Die Erfindung betrifft weiter die Verwendung der Zusammensetzung in der Therapie verschiedener Schilddrüsenerkrankungen, zur Verringerung der Nebenwirkungen resultierend aus der Verabreichung von Schilddrüsenhormonen und die Verwendung in der Suppressions- und Substitutionstherapie bei Schilddrüsenmalignom.

## Beschreibung

Die vorliegende Erfindung betrifft eine pharmazeutische Zusammensetzung, welche ein Schilddrüsenhormon und eine Selenverbindung enthält, sowie die Verwendung der Zusammensetzung in der Therapie verschiedener Schilddrüsenerkrankungen.

Zur Therapie verschiedener Störungen der Schilddrüsenfunktion werden Schilddrüsenhormone eingesetzt. Die Gabe des Schilddrüsenhormons L-Thyroxin verursacht bei mindestens 20 % der Patienten bis zu 17 unerwünschte Nebenwirkungen (Quelle: ABDA Datenbank vom 22.02.2017). Schlafstörungen, Gefühlsschwankungen und übermäßige Schweißbildung gehören zu den häufigsten Nebenwirkungen.

Eine Aufgabe der Erfindung war die Entwicklung eines neuen Schilddrüsenmedikaments mit verbesserter Wirkung und weniger Nebenwirkungen.

Es wurde nun gefunden, dass diese Aufgabe gelöst wird durch ein Kombinationspräparat, welches mindestens ein Schilddrüsenhormon und mindestens eine Selenverbindung in einem definierten Mengenverhältnis umfasst.

Gegenstand der Erfindung ist daher eine pharmazeutische Zusammensetzung, welche L-Thyroxin und mindestens eine Selenverbindung enthält, wobei das Gewichtsverhältnis von L-Thyroxin zu Selen von 2:1 bis 1:2 beträgt. Das Gewichtsverhältnis bezieht sich dabei auf die in der mindestens einen Selenverbindung enthaltene Menge Selen, also nicht auf das Gesamtgewicht der mindestens einen Selenverbindung.

Der Begriff "pharmazeutische Zusammensetzung" bedeutet, dass die Zusammensetzung als Medikament eingesetzt wird.

In einer Ausführungsform der erfindungsgemäßen pharmazeutischen Zusammensetzung beträgt das Gewichtsverhältnis von L-Thyroxin zu Selen von 1,5:1 bis 1:1,5. In einer anderen Ausführungsform der erfindungsgemäßen pharmazeutischen Zusammensetzung beträgt das Gewichtsverhältnis von L-Thyroxin zu Selen von 1,4:1 bis 1:1,4. In einer weiteren Ausführungsform der erfindungsgemäßen pharmazeutischen Zusammensetzung beträgt das Gewichtsverhältnis von L-Thyroxin zu Selen von 1,3:1 bis 1:1,3. In einer anderen Ausführungsform der erfindungsgemäßen pharmazeutischen Zusammensetzung beträgt das Gewichtsverhältnis von L-Thyroxin zu Selen von 1,2:1 bis 1:1,2.In einer weiteren Ausführungsform der erfindungsgemäßen pharmazeutischen Zusammensetzung beträgt das Gewichtsverhältnis von L-Thyroxin zu Selen von 1,1:1 bis 1:1,1. In einer anderen Ausführungsform der erfindungsgemäßen pharmazeutischen Zusammensetzung beträgt das Gewichtsverhältnis von L-Thyroxin zu Selen 1:1.

Die erfindungsgemäße pharmazeutische Zusammensetzung enthält L-Thyroxin (T₄, 3,3',5,5'-Tetraiod-L-thyronin).

In einer Ausführungsform enthält die erfindungsgemäße pharmazeutische Zusammensetzung als Selenverbindung Natriumselenit. In einer anderen Ausführungsform enthält die erfindungsgemäße pharmazeutische Zusammensetzung als Selenverbindung Natriumselenat.

In einer anderen Ausführungsform enthält die erfindungsgemäße pharmazeutische Zusammensetzung als Selenverbindung eine Selenoaminosäure. In einer weiteren Ausführungsform enthält die erfindungsgemäße pharmazeutische Zusammensetzung als Selenverbindung L-Selenomethionin. In einer weiteren Ausführungsform enthält die erfindungsgemäße pharmazeutische Zusammensetzung als Selenverbindung L-Selenocystein.

Die erfindungsgemäße pharmazeutische Zusammensetzung kann auch Mischungen der genannten Selenverbindungen enthalten.

Gegenstand der Erfindung ist auch die erfindungsgemäße Zusammensetzung zur Verringerung der Nebenwirkungen der Verabreichung von Schilddrüsenhormonen. Insbesondere werden durch die erfindungsgemäße Zusammensetzung als häufige Nebenwirkungen der Hormontherapie auftretende Schlafstörungen, übermäßige Schweißbildung und Stimmungsschwankungen verringert oder gänzlich beseitigt.

Gegenstand der Erfindung ist auch die erfindungsgemäße Zusammensetzung zur Therapie von Hypothyreose.

Gegenstand der Erfindung ist auch die erfindungsgemäße Zusammensetzung zur Prophylaxe einer Rezidivstruma nach Resektion einer Struma mit euthyreoter Funktionslage.

Gegenstand der Erfindung ist auch die erfindungsgemäße Zusammensetzung zur Therapie einer benignen Struma mit euthyreoter Funktionslage.

Gegenstand der Erfindung ist auch die erfindungsgemäße Zusammensetzung zur Begleittherapie bei thyreostatischer Behandlung einer Hyperthyreose nach Erreichen der euthyreoten Funktionslage.

Gegenstand der Erfindung ist auch die erfindungsgemäße Zusammensetzung zur Suppressions- und Substitutionstherapie bei Schilddrüsenmalignom, insbesondere nach Thyreoidektomie. Ein weiterer Gegenstand der Erfindung ist die erfindungsgemäße Zusammensetzung in Kombination mit Jod zur Suppressions- und Substitutionstherapie bei Schilddrüsenmalignom, insbesondere nach Thyreoidektomie.

Gegenstand der Erfindung ist auch die erfindungsgemäße Zusammensetzung zur Therapie von Schilddrüsenentzündungen vom Hashimoto-Typ.

Gegenstand der Erfindung ist auch die erfindungsgemäße Zusammensetzung zur Therapie von Schlafstörungen.

Gegenstand der Erfindung ist auch die erfindungsgemäße Zusammensetzung zur Therapie übermäßiger Schweißproduktion.

Gegenstand der Erfindung ist auch die erfindungsgemäße Zusammensetzung zur Therapie von Stimmungsschwankungen.

In einer Ausführungsform wird die erfindungsgemäße Zusammensetzung in einer Dosierung eingesetzt, bei der dem Patienten pro Tag jeweils im Bereich von 0,5 bis 2 µg pro kg Körpergewicht L-Thyroxin und die entsprechende in der Zusammensetzung enthaltene Menge Selen zugeführt werden. Die Dosierung beträgt beispielsweise 0,5 bis 1,5 µg, oder 0,5 bis 1 µg. In einem Beispiel erhält ein 100 kg schwerer Mensch täglich eine Tablette, die 100 µg L-Thyroxin und 100 µg Natriumselenit als Selen berechnet enthält, also 219 µg Natriumselenit (Gewichtsverhältnis L-Thyroxin/Selen 1:1).

In einer Ausführungsform enthält eine Dosis der erfindungsgemäßen Zusammensetzung 50 µg L-Thyroxin und 50 µg Selen. In einer anderen Ausführungsform enthält eine Dosis der erfindungsgemäßen Zusammensetzung 75 µg L-Thyroxin und 75 µg Selen. In einer anderen Ausführungsform enthält eine Dosis der erfindungsgemäßen Zusammensetzung 88 µg L-Thyroxin und 88 µg Selen. In einer anderen Ausführungsform enthält eine Dosis der erfindungsgemäßen Zusammensetzung 100 µg L-Thyroxin und 100 µg Selen. In einer anderen Ausführungsform enthält eine Dosis der erfindungsgemäßen Zusammensetzung 112 µg L-Thyroxin und 112 µg Selen. In einer anderen Ausführungsform enthält eine Dosis der erfindungsgemäßen Zusammensetzung 125 µg L-Thyroxin und 125 µg Selen. In einer anderen Ausführungsform enthält eine Dosis der erfindungsgemäßen Zusammensetzung 137,5 µg L-Thyroxin und 137,5 µg Selen. In einer anderen Ausführungsform enthält eine Dosis der erfindungsgemäßen Zusammensetzung 150 µg L-Thyroxin und 150 µg Selen. In einer anderen Ausführungsform enthält eine Dosis der erfindungsgemäßen Zusammensetzung 175 µg L-Thyroxin und 175 µg Selen.

In einer Ausführungsform enthält die erfindungsgemäße Zusammensetzung zusätzlich Jod. In einer weiteren Ausführungsform beträgt die Menge an Jod 1,5 µg pro kg Körpergewicht des Patienten. In einer Ausführungsform enthält die erfindungsgemäße Zusammensetzung das Jod in Form von Natriumiodid. In einer weiteren Ausführungsform beträgt das Gewichtsverhältnis von L-Thyroxin, Selen und Jod in der erfindungsgemäßen Zusammensetzung 1:1:1.

In einer Ausführungsform enthält die erfindungsgemäße Zusammensetzung zusätzlich L-Tyrosin. In einer weiteren Ausführungsform enthält eine Dosis der erfindungsgemäßen Zusammensetzung 500 mg L-Tyrosin.

In einer Ausführungsform enthält die erfindungsgemäße Zusammensetzung zusätzlich Vitamin D (Vitamin D₃, 3-[2-[7a-Methyl-1-(6-methylheptan-2-yl)-2,3,3a,5,6,7-hexahydro-1H-inden-4-yliden]ethyliden]-4-methyliden-cyclohexan-1-ol). In einer weiteren Ausführungsform enthält eine Dosis der erfindungsgemäßen Zusammensetzung 50 i. E. Vitamin D.

In einer Ausführungsform enthält die erfindungsgemäße Zusammensetzung zusätzlich Vitamin C (Ascorbinsäure). In einer weiteren Ausführungsform enthält eine Dosis der erfindungsgemäßen Zusammensetzung 500 mg Vitamin C.

In einer Ausführungsform enthält die erfindungsgemäße Zusammensetzung L-Thyroxin, Selen und Jod. In einer anderen Ausführungsform enthält die erfindungsgemäße Zusammensetzung L-Thyroxin, Selen und L-Tyrosin. In einer anderen Ausführungsform enthält die erfindungsgemäße Zusammensetzung L-Thyroxin, Selen und Vitamin D. In einer anderen Ausführungsform enthält die erfindungsgemäße Zusammensetzung L-Thyroxin, Selen und Vitamin C. In einer weiteren Ausführungsform enthält die erfindungsgemäße Zusammensetzung L-Thyroxin, Selen, lod und L-Tyrosin. In einer weiteren Ausführungsform enthält die erfindungsgemäße Zusammensetzung L-Thyroxin, Selen und lod sowie Vitamin D und/oder Vitamin C. In einer weiteren Ausführungsform enthält die erfindungsgemäße Zusammensetzung L-Thyroxin, Selen und L-Tyrosin sowie Vitamin D und/oder Vitamin C. In einer weiteren Ausführungsform enthält die erfindungsgemäße Zusammensetzung L-Thyroxin, Selen, lod und L-Tyrosin sowie Vitamin D und/oder Vitamin C.

Gegenstand der Erfindung ist auch die erfindungsgemäße Zusammensetzung zur Verabreichung in einer Dosierung von 0,5 bis 2 µg pro kg Körpergewicht des Patienten und Tag, bezogen auf L-Thyroxin. In einer Ausführungsform beträgt die Dosierung 1,5 µg pro kg Körpergewicht des Patienten und Tag, bezogen auf L-Thyroxin. In einer weiteren Ausführungsform beträgt die Dosierung 1,5 µg pro kg Körpergewicht des Patienten und Tag, bezogen auf L-Thyroxin und Selen.

Studien an 100 Patienten zeigten, dass bei Verabreichung eines 100 µg L-Thyroxin und 200 µg Selen enthaltenden Kombinationspräparats im Vergleich zu ausschließlicher Gabe von L-Thyroxin weniger Nebenwirkungen auftraten.

In den Studien verschwanden bei allen Patienten Nebenwirkungen des L-Thyroxins wie Schlafstörungen, Gefühlsschwankungen, Schwindelgefühl, Unruhe und übermäßige Schweißbildung komplett innerhalb von 4 Tagen.

Auch Symptome wie Herzrasen, Herzrhythmusstörungen, Herzklopfen und Panikattacken traten nach Umstellung auf das Kombinationspräparat nicht mehr auf.

Außerdem verschwanden durch die Gabe des Kombinationspräparats in drei Fällen auch Schilddrüsenentzündungen vom Hashimoto-Typ.

In 500 Fällen berichteten Patienten, dass sie bei Einnahme des Kombinationspräparates mehr Energie hätten als bei Einnahme des Schilddrüsenhormons allein.

## Patentansprüche

1. Zusammensetzung enthaltend L-Thyroxin und mindestens eine Selenverbindung, wobei das Gewichtsverhältnis von L-Thyroxin zu Selen in der Selenverbindung von 2:1 bis 1:2 beträgt, als Medikament.

2. Zusammensetzung nach Anspruch 1, bei der das Gewichtsverhältnis von L-Thyroxin zu Selen in der Selenverbindung 1:1 beträgt.

3. Zusammensetzung nach Anspruch 1 oder 2, bei der die mindestens eine Selenverbindung Natriumselenit ist.

4. Zusammensetzung nach einem der Ansprüche 1 bis 3, welche zusätzlich lod enthält.

5. Zusammensetzung nach einem der Ansprüche 1 bis 4, welche zusätzlich L-Tyrosin enthält.

6. Zusammensetzung nach einem der Ansprüche 1 bis 5, welche zusätzlich Vitamin D enthält.

7. Zusammensetzung nach einem der Ansprüche 1 bis 6, welche zusätzlich Vitamin C enthält.

8. Zusammensetzung nach einem der Ansprüche 1 bis 7 zur Verringerung der Nebenwirkungen der Verabreichung von Schilddrüsenhormonen.

9. Zusammensetzung nach einem der Ansprüche 1 bis 7 zur Therapie von Hypothyreose.

10. Zusammensetzung nach einem der Ansprüche 1 bis 7 zur Prophylaxe einer Rezidivstruma nach Resektion einer Struma mit euthyreoter Funktionslage.

11. Zusammensetzung nach einem der Ansprüche 1 bis 7 zur Therapie einer benignen Struma mit euthyreoter Funktionslage.

12. Zusammensetzung nach einem der Ansprüche 1 bis 7 zur Begleittherapie bei thyreostatischer Behandlung einer Hyperthyreose nach Erreichen der euthyreoten Funktionslage.

13. Zusammensetzung nach einem der Ansprüche 1 bis 7 zur Suppressions- und Substitutionstherapie bei Schilddrüsenmalignom , insbesondere nach Thyreoidektomie.

14. Zusammensetzung nach einem der Ansprüche 1 bis 7 in Kombination mit Jod zur Suppressions- und Substitutionstherapie bei Schilddrüsenmalignom , insbesondere nach Thyreoidektomie.

15. Zusammensetzung nach einem der Ansprüche 1 bis 7 zur Therapie von Schilddrüsenentzündungen vom Hashimoto-Typ.
